# EUROPEAN PATENT SPECIFICATION

(11) **EP 1 837 054 B1**
(45) Date of publication and mention of the grant of the patent: **29.06.2011**
(21) Application number: 06005854.2
(22) Date of filing: 22.03.2006
(51) Int. Cl.: A61Q 19/00, A61K 8/67, A61K 8/97

(54) **Cosmetic composition comprising seabuckthorn**
Kosmetische Zusammensetzung enthaltend Sanddorn
Compositions cosmétiques comprenant de l'argousier

(43) Date of publication of application: 26.09.2007
(73) Proprietor: The Procter & Gamble Company, Cincinnati, OH 45202 (US)
(72) Inventor: Gibbons, Clyde, Staines Middlesex TW18 3DE (GB); Watson, Andrew David, Meadvale Redhill Surrey, RH1 6ND (GB); Morris, Sian, Bracknell Berkshire, RG12 0UZ (GB)
(74) Representative: Wilding, Richard Alan

(56) References cited:
- FR-A- 2 840 809
- US-A1- 2001 036 468
- DATABASE WPI Section Ch, Week 198221 Derwent Publications Ltd., London, GB; Class D21, AN 43410E XP002374412 & SU 852 331 B (ALEKSANYAN T I) 7 August 1981 (1981-08-07)
- DATABASE WPI Section Ch, Week 199817 Derwent Publications Ltd., London, GB; Class B05, AN 191538 XP002232119 & RO 112 158 A (CALINESCU B C) 30 June 1997 (1997-06-30)
- DATABASE WPI Week 199630 Derwent Publications Ltd., London, GB; AN 298696 XP000232120 & RU 2 048 802 A (DOLOTOVSKAYA L Z) 27 November 1995 (1995-11-27)
- YANG B ET AL: "Composition and physiological effects of sea buckthorn (Hippophae) lipids" TRENDS IN FOOD SCIENCE AND TECHNOLOGY, ELSEVIER SCIENCE PUBLISHERS, GB, vol. 13, no. 5, May 2002 (2002-05), pages 160-167, XP004394588 ISSN: 0924-2244

## Description

### FIELD OF THE INVENTION

The present invention relates to cosmetic topical compositions comprising a hydrophilic extract of Seabuckthorn, especially Seabuckthorn berries in combination with 0.01% to 10% of one or more hydrophilic vitamins, by weight of the composition. Seabuckthorn is a shrub having the latin name *"Hippophae Rhamnoides",* which is native to Eurasia.

### BACKGROUND OF THE INVENTION

Extract of Seabuckthorn berries have been used for thousands of years, in ancient Greece and Rome, even in Tibetan monasteries. References to the medicinal use of Seabuckthorn have been found in both Ancient Greek texts attributed to Theophrastus and Dioskorid and in classic Tibetan medicinal texts including 'the RGyud Bzi' (the Four Books of Pharmacopoeia) dated to the times of Tang Dynasty (618-907 AD). Today it is mainly used in Scandinavia, Eastern Europe (Russia) and China.

Hydrophobic Seabuckthorn extract, or Seabuckthorn oil, is used in cosmetics and medicine to promote the regeneration of the skin and mucous membranes (e.g. burns, healing wounds, skin damaging effect of sun, eczema) and to regulate and/or to promote the recovery of various skin conditions due to revitalizing, nourishing and anti-ageing properties. Chinese patent application 1195489, Rumanian patent applications 112158, 63707, 62717 and 62721 , Japanese patent application 2-108613 and Russian patent application 852331 all relate to extracts of Seabuckthorn for use on skin or hair. The beneficial properties of Seabuckthorn oil appear to derive from a number of components, such as fatty acids, carotenes, tocopherols, and phytosterol. Nevertheless, in spite of its properties, the use of Seabuckhorn oil in the cosmetic field has been limited by its highly red colour, its strong, unpleasant odour, its negative impact on the tackiness of compositions and its poor feel following application to skin.

Hydrophilic Seabuckthorn extract has also been used in cosmetics, in particular for its antioxidant and anti-ageing properties. Russian patent 2 048 802 teaches that aqueous-alcoholic-glycerine extracts of Seabuckthorn in combination with other botanical extracts provides skin anti-ageing and other benefits. Lastly, French patent applications 2 840 808 and 2 840 809 teach the use of hydrophilic fractions of Seabuckthorn for preventing and/or treating the dermis against the ageing of the skin, e.g. by inhibiting hyaluronidase, elastase and collagenase. Patents SU 852331 and RO112158 also relate to the cosmetic use of hydrophilic Seabuckthorn extracts.

Hydrophilic vitamin components are also known in the cosmetic field for their various properties, such as skin-conditioning. Nevertheless, many hydrophilic vitamin components cannot be formulated within cosmetic compositions or can only be formulated at low concentrations, because they are unstable or cause skin irritation.

In order to improve safety, stability, quality, efficacy and/or the spectrum of activity of cosmetic compositions, there is a constant need to develop compositions exhibiting improved properties which do not have negative side effects. More precisely, there is a need to deliver long term and gradual improvements to the skin, these improvements serving to give skin a natural and even tone, to provide improved skin texture, and to help enhance the skin's natural barrier function.

Unexpectedly, the inventors have found that the effects of a hydrophilic extract of Seabuckthorn on skin tone, skin texture, and skin barrier function can be synergistically improved by combining it with at least one hydrophilic vitamin component.

### SUMMARY OF THE INVENTION

According to a first aspect of the invention, cosmetic compositions according to claim 1 comprising a combination of a hydrophilic extract of Seabuckthorn and a hydrophilic vitamin component are provided.

The present inventors have now surprisingly found that hydrophilic extracts of Seabuckthorn in combination with hydrophilic vitamin component may give rise to unexpected benefits when used in the cosmetic treatment of skin. Thus, said combination may show synergistic effects on regulating the skin condition, and in particular on improving skin tone, skin texture and skin barrier function. Without wishing to be limited by theory, applicants believe that the synergistic effects may be obtained by the action of the hydrophilic extract of Seabuckthorn and the hydrophilic vitamin component both on the protective and maintenance mechanisms of the skin. More precisely, it is believed that the synergistic effects may be obtained by the potentialisation of the effects of the hydrophilic extract of Seabuckthorn by the hydrophilic vitamin components. The hydrophilic vitamin components may potentiate the effect produced by the hydrophilic extract of Seabuckthorn by acting on the same biochemical mechanisms as the extract and/or the hydrophilic vitamin components may act on biochemical mechanisms different for those targeted by the hydrophilic extract of Seabuckthorn.

### DEFINITIONS

As used herein, the word "hydrophilic" in relation to a material means that that material is more than 10% soluble in water by weight at standard temperature and pressure (STP).

As used herein, the word "hydrophobic" as used in relation to a material means that that material is less than 0.1% soluble in water by weight at standard temperature and pressure (STP).

### DETAILED DESCRIPTION OF THE INVENTION

Cosmetic compositions according to the invention comprise a hydrophilic extract of Seabuckthorn. Any extract of any part of the Seabuckthorn plant, which is hydrophilic, as defined herein, is suitable for incorporation in the present composition.

Preferably, the extract is derived from Seabuckthorn berries. Suitably, in order to produce the present extract, ripe Seabuckthorn berries are harvested, cleaned and comminuted. The juice is obtained by cold pressing of the berry pulp to obtain the juice, filtered then concentrated by low temperature distillation, after which it may be diluted with hydrophilic diluents, such as water, poylols, alcohols and other additives. A suitable product containing a hydrophilic extract of Seabuckthorn is commercially available under the name FRUITAPONE® SEABUCKTHORN B from Symrise (INCI name: propylene glycol, aqua, citric acid, Hippophae Rhamnoides, trideceth-9, bisabolol), said product comprising 5% of hydrophilic extract of Seabuckthorn.

The cosmetic composition may comprise from 0.0001% to 1%, preferably from 0.001% to 0.4%, and more preferably from 0.002% to 0.15% of a hydrophilic extract of Seabuckthorn by weight of the composition.

Cosmetic compositions according to the invention further comprise a hydrophilic vitamin component. The hydrophilic vitamin component may comprise any vitamin or derivative thereof that fulfils the definition of "hydrophilic" provided herein. Preferably, the hydrophilic vitamin component comprises vitamin B₃, salts or esters thereof; pro-vitamin B₅, salts or esters thereof; vitamin C, salts or esters thereof; hydrophilic derivatives of vitamin E, such as tetramethylchromanol glucosides and 6-hydroxy-2,5,7,8-tetramethylchroman-2-carboxylic acid (commercially available as TROLOX^{™}); or mixtures thereof. Preferably, the hydrophilic vitamin component comprises vitamin B₃, salts or esters thereof; vitamin B₅, salts or esters thereof, or mixtures thereof More preferably, the hydrophilic vitamin component comprises vitamin B₃, salts or esters thereof, or mixtures thereof.

Without wishing to be bound by theory, it is believed that hydrophilic vitamin components may synergistically potentiate the effect of the hydrophilic extract of Seabuckthorn on skin tone, skin texture, skin hydratation and skin barrier function. Among other things, applicants believe that the hydrophilic vitamin components may synergistically enhance the effects of a hydrophilic extract of Seabuckthorn on tyrosinase inhibition, hyaluronic acid up-regulation, pro-collagen and keratin up-regulation, COX-I and COX-II anti-inflammation mediation and/or as free radicals scavenger (antioxidant properties).

The cosmetic composition according to the invention comprises from 0.01% to 10%, preferably from 0.1% to 9%, more preferably from 0.5% to 5% of hydrophilic vitamin component by weight of the composition.

Advantageously, the cosmetic compositions according to the invention comprise a combination of a hydrophilic extract of seabuckthorn and niacinamide.

Cosmetic compositions according to the invention may further comprise at least one humectant. Preferably, the humectant comprises polyhydric alcohols. More preferably, the humectant comprises glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin or mixtures thereof. Still more preferably, the humectant comprises glycerine. The cosmetic composition according to the invention may comprise from 0.1 to 30%, preferably from 5% to 25% and more preferably from 7% to 15% humectant by weight of the cosmetic composition.

Cosmetic compositions according to the present invention may further comprise at least one thickener. Thickeners which may be employed according to the invention comprise C₁₂-C₂₄ fatty acids; C₁₀-C₃₀ fatty alcohols; N-fatty glutamic acid dialylamides; carboxy methyl cellulose or derivatives thereof; polysaccharide gums, such as xanthan gum and guar gum; starch or derivatives thereof; particulate thickeners, such as hydrated colloidal aluminium silicate and magnesium aluminium silicate; carboxyvinyl polymers; sodium acrylate copolymers and hydrophobically modified sodium acrylate copolymers; polyacrylamide copolymers; polyacrylates, such as polyacrylate-13; acrylamide/ammonium acrylate copolymer; ammonium acryloyldimethyl taurate and ammonium acryloyldimethyl taurate crosspolymers and copolymers and hydrophobically modified ammonium acryloyldimethyl taurate copolymers; cationionically charged polymers, such as polyquaternium polymers and polyquaternium copolymers; or mixtures thereof. Preferably, the thickener comprises Luvigel® EM (BASF Corporation), which is a milky emulsion of sodium acrylates copolymer in caprylic/capric triglyceride and water (INCI name: caprylic/capric triglyceride (and) sodium acrylates copolymer). Luvigel® EM is provided in the form of a water-in-oil emulsion which inverts on addition to water. It is preferred because it may significantly reduce the tack or stickiness when topically applied to the skin. The cosmetic composition according to the invention may comprise from 0.01% to 10%, preferably from 0.1% to 7% and more preferably from 1% to 3% thickener by weight of the composition.

Cosmetic compositions according to the present invention may further, comprise an additional hydrophilic botanical component. The additional hydrophilic botanical component may comprise any botanical ingredient that fulfils the definition of "hydrophilic" provided herein. The additional hydrophilic botanical component may advantageously comprise an hydrophilic extract of aloe vera leaf *(aloe vera),* argan tree leaf and/or fruit *(argania spinosa),* baobab or Cream of Tartar tree leaf *(adansonia digitata),* bearberry leaf *(arctostaphylos),* brahmi (or water hyssop) whole plant *(Bacopa monnieri),* butcher's broom *(ruscus root),* centella asiatica *(Centella asiatica),* chamomile *(Chamomilla Recuita matricaria),* chestnut *(castanea sativa),* clary sage leaf *(salvia sclarea),* eyebright leaf *(euphrasia officinalis),* ginkgo biloba leaf *(ginkgo biloba),* grape fruit, leaf and/or seed *(vitis vinifera),* green tea leaf *(Camellia Sinensis),* honey *(mel),* horestail leaf *(equisetum arvense),* horse chestnut seed *(aesculus hippocastanum),* Indian cress whole plant *(tropaelolum ajus),* lemongrass plant leaf *(cymbopogon Schoenanthus),* linden wood, leaf and/or bark *(Tilia Cordata),* liquorice root *(glycyrrhiza glabra),* marigold flower *(calendula officinalis),* mimosa leaf and/or bark *(mimosa tenuiflora),* mulberry leaf *(morus nigra),* neem leaf and/or bark *(azadirachta indica),* nettle leaf *(urtica dioica),* oat germ and/or bran *(Avena sativa),* oergano leaf *(Origanum officinalis),* olive leaf and/or fruit *(Olea Europaea),* panax ginseng root extract *(Panax Ginseng),* plantago leaf *(plantago lanceolata),* propolis *(propolis),* rose hip fruit *(Rosa canina),* rosemary leaf *(Rosmarinus),* sage leaf *(salvia officinalis),* st. Mary's thisle *(silybum marianum),* sesame seed *(Sesamum indicum),* sweet manadarin peel *(citrus nobilis),* wheat bran and/or germ *(triticum vulgare),* willow bark *(Salix alba),* witch hazel *(hamamelis Virginia)* or mixtures thereof, preferably rose hip fruit, ginko biloba leaf, panax ginseng root or mixtures thereof, more preferably rose hip fruit.

Without wishing to be bound by any theory, it is believed for example that the combination of a hydrophilic extract of Seabuckthorn and a hydrophilic vitamin with an additional hydrophilic botanical component, preferably a hydrophilic extract of rose hip, may enhance the synergistic effect obtained by the combination of the hydrophilic extract of Seabuckthorn and the hydrophilic vitamin alone.

The cosmetic composition according to the invention may comprise from 0.0001 % to 1%, preferably from 0.001% to 0.4% and more preferably from 0.002% to 0.15% of the additional hydrophilic botanical component by weight of the composition.

Cosmetic compositions according to the present invention may further comprise an additional hydrophobic botanical component. The additional hydrophobic botanical component may comprise any botanical ingredient that fulfils the definition of "hydrophobic" provided herein. The additional hydrophobic botanical component may advantageously comprise argan oil *(Argania Spinosa),* bilberry seed oil *(Vaccinium myrtihus),* blackcurrant seed oil *(Ribes Nigrum),* cotton seed oil *(Gossypium Herbaceum),* cranberry seed oil *(Vaccinium Macrocarpon),* cumin extract (comprising tetrahydrocurcumin), evening primrose oil *(Oenothera Biennis),* grape seed oil *(Vitis Vinifera),* grapefruit seed oil *(Citrus Grandis),* kiwi seed oil *(Actinidia Chinensis),* lavender *(Lavendular Augustafolia),* ligonberry seed oil *(Vaccinium Vitis-Idaea),* lime seed oil *(Citrus auranifolia),* linseed oil *(Linum usitatissimum),* liquorice root *(glycyrrhiza glabrai),* olive oil *(Olea Europaea),* olive wax *(Olea Europaea),* organic grape seed oil *(Vitis Vinifera),* oriental popy seed oil *(Papaver Orientale),* meadowfoam seed oil *(Limnanthes alba),* neem leaf and/or bark oil *(azadirachta indica),* palm oil *(Elaeis guineenis),* papaya seed oil *(Carica Papaya),* passion fruit seed oil *(Passiflora edulis),* pumpkin seed oil *(Cuerbita pepo),* raspberry seed oil *(Rubus idaeus),* rosehip seed oil *(Rosa Canina),* rosemary oil *(Rosmarinus officinalis),* seabuckthorn seed oil *(Hippophae Rhamnoides),* sesame seed oil *(Sesamum indicum),* shea butter *(butyrospermum parkii),* soy extract, sweet almond oil *(Prunus amygdalus dulcis),* watermelon seed oil *(Citrullus vulgaris),* or mixtures thereof. The cosmetic composition according to the invention may comprise from 0.0001% to 1%, preferably from 0.001 % to 0.4%, and more preferably from 0.002% to 0.15% of an additional hydrophobic botanical component by weight of the composition.

Cosmetic compositions according to the present invention may further comprise a hydrophobic vitamin component. The hydrophobic vitamin component may comprise any vitamin that fulfils the definition of "hydrophobic" provided herein. The hydrophobic vitamin component may advantageously comprise vitamin E or hydrophobic derivatives thereof, preferably tocophetyl nicotamide, vitamin D or derivatives thereof, or mixtures thereof. The cosmetic composition according to the invention may comprise from 0.01% to 10%, preferably from 0.1% to 9%, more preferably from 0.5% to 5% of at least one hydrophobic vitamin component by weight of the composition.

Cosmetic compositions according to the present invention may further comprise at least one sunscreen active, which may be a hydrophobic organic sunscreen active, a hydrophilic organic sunscreen active, an inorganic sunscreen, or mixtures thereof. Suitable examples of sunscreens may be found in the CTFA International Cosmetic Ingredient Dictionary and Handbook, 7th edition, volume 2 pp. 1672, edited by Wenninger and McEwen (The Cosmetic, Toiletry, and Fragrance Association, Inc., Washington, D. C., 1997).

Preferred hydrophobic organic suncreens suitable to be included in compositions according to the invention comprise alkyl β,β-diphenylacrylate and/or α-cyano β,β-diphenylacrylate derivatives, such as ethyl 2-cyano-3,3-diphenylacrylate, 2-ethylhexyl 2-cyano-3,3-diphenylacrylate (octocrylene); dibenzoylmethane derivatives, such as butyl methoxydibenzoylmethane (avobenzone), ethylhexyl methoxydibenzoylmethane, isopropyl dibenzoylmethane; benzophenone derivatives, such as benzophenone-1 (benzoresorcinol benzoresorcinol), benzophenone-2 (tetraydroxybenzophenone), benzophenone-3 (oxybenzone), benzophenone-4 (sulisobenzone), benzophenone-5 (sulisobenzone sodium), benzophenone-6 (dihydroxy dimethoxy benzophenone), benzophenone-7 (2-benzoyl-4-chlorophenol), benzophenone (8 dioxybenzone), benzophenone-9, benzophenone-10 (mexenone), benzophenone-11, benzophenone-12 (octabenzone); cinnamic derivatives, such as octyl methoxycinnamate (octinoxate), diethanolamine methoxycinnamate; salicylic derivatives, such as ethylhexyl salicylate (octisalate), triethanolamine salicylate, 3,3,5-trimethylcyclohexylsalicylate, homomenthyl salicylate (homosalate); camphor derivatives, such as 4-methylbenzylidene camphor (enzacamene); triazine derivatives, such hexylethyl triazone; anthranilate derivatives, such as menthyl anthranilate (meradimate); p-aminobenzoic acid derivatives, such as aminobenzoic acid (PABA), glyceryl PABA (lisadimate), octyl dimethyl PABA, ethyl dihydroxypropyl PABA; or mixture thereof More preferably, hydrophobic organic suncreens comprise alkyl β,β-diphenylacrylate and/or α-cyano β,β-diphenylacrylate derivatives, such as ethyl 2-cyano-3,3-diphenylacrylate, 2-ethylhexyl 2-cyano-3,3-diphenylacrylate; dibenzoylmethane derivatives, such as butyl methoxydibenzoylmethane, ethylhexyl methoxydibenzoylmethane, isopropyl dibenzoylmethane. Still more preferably, hydrophobic organic sunscreens comprise 2-ethylhexyl 2-cyano-3,3-diphenylacrylate, butyl methoxydibenzoylmethane, or mixtures thereof. Cosmetic compositions according to the invention may comprise from 0.01% to 15%, preferably from 0.1% to 14%, and more preferably from 1% to 8% of a hydrophobic organic sunscreen by weight of the composition.

A preferred hydrophilic organic suncreens suitable to be included in compositions according to the invention comprises 2-phenylbenzimidazole-5-sulfonic acid (PBSA). The cosmetic composition according to the invention may comprise from 0.1% to 5%, preferably from 0.5% to 2.25%, and more preferably from 0.75% to 1.5% of a hydrophilic organic sunscreen by weight of the composition.

Cosmetic compositions according to the present invention may additionally comprise at least one inorganic sunscreen active having a mean primary particle size less than to 200 nm, preferably less than 100 nm, and most preferably from 1 to 30 nm. Preferably, inorganic sunscreen actives comprise titanium dioxide, zinc oxide, or mixtures thereof The cosmetic composition according to the invention may comprise from 1% to 15%, preferably from 1% to 10% of an inorganic sunscreen by weight of the composition.

Cosmetic compositions according to the present invention may further comprise at least one tanning active. Preferably, said tanning active is selected from dihydroxyacetone (DHA), salts, derivatives or tautomers thereof, or mixtures thereof. The cosmetic composition according to the invention may comprise from 0.01% to 10%, preferably from 0.05% to 5%, and more preferably from 0.1% to 1% of at least one tanning active by weight of the composition.

Cosmetic compositions according to the present invention may further comprise at least one sugar amine. Said sugar amine can be synthetic or natural in origin and can be used as pure compounds or mixtures of compounds (e.g., extracts from natural sources or mixtures of synthetic materials). Preferably, sugar amine comprises glucosamine, N-acetyl glucosamine, mannosamine, N-acetyl mannosamine, galactosamine, N-acetyl galactosamine, isomers thereof (e.g., stereoisomers), salts thereof (e.g., HCl salt), or mixtures thereof. The cosmetic composition according to the invention may comprise from 0.01% to 15%, preferably from 0.1% to 10%, and more preferably from 0.5% to 5% of at least one sugar amine by weight of the composition.

Cosmetic compositions according to the present invention may further comprise at least one hexaminidine compound, salts or derivatives thereof, or mixtures thereof. Preferably, hexaminidine compound comprises compounds corresponding to the following chemical structure: 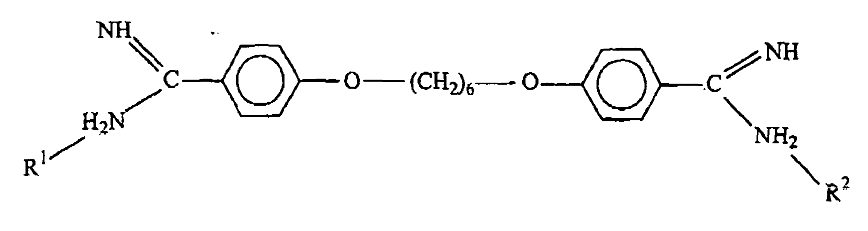 wherein R¹ and R² are organic acids (e.g., sulfonic acids, etc.). The cosmetic composition according to the invention may comprise from 0.001 % to 10%, preferably from 0.01% to 5%, and more preferably from 0.02% to 2.5% of a hexaminidine compound by weight of the composition.

The cosmetic composition according to the invention may be in the form of an emulsion, e.g. water-in-oil or oil-in-water emulsion, or in a form of micellar surfactant/detergent system (e.g. cleansing milk, face wash). Advantageously, the cosmetic composition according to the invention is in the form of an oil-in-water emulsion.

The cosmetic composition in the form of an oil-in-water emulsion may further comprise at least one hydrophilic surfactant. Generally, hydrophilic surfactants help disperse and suspend the discontinuous phase within the continuous phase. Preferably, hydrophilic surfactants are selected from nonionic surfactants such as those known in the art. Other suitable surfactants useful herein comprise a wide variety of cationic, anionic, zwitterionic, and amphoteric surfactants such as are known in the art. See, e.g., McCutcheon's, Detergents and Emulsifiers, North American Edition (1986), published by Allured Publishing Corporation. The hydrophilic surfactants useful herein can contain a single surfactant, or any combination of suitable surfactants. The exact surfactant (or surfactants) chosen will depend upon the pH of the composition and the other components present. The cosmetic composition according to the invention may comprise from 0.05% to 10%, preferably from 1% to 6%, and more preferably from 1% to 3% of at least one hydrophilic surfactant by weight of the composition.

The oil phase may comprise at least one oily component such as a natural or synthetic oils selected from mineral, vegetable, and animal oils, fats and waxes, fatty acid esters, fatty alcohols, fatty acids, or mixtures thereof. For example, the oil phase may comprise saturated and unsaturated fatty alcohols such as behenyl alcohol, cetyl alcohol and stearyl alcohol and hydrocarbons such as mineral oils or petrolatum.

Cosmetic compositions of the present invention may further comprise at least one emollient material including branched chain hydrocarbons having a weight average molecular weight of from 100 to 15,000, preferably from 100 to 1000. Preferably branched chain hydrocarbons comprise isododecane, isohexadecane, isoeicosane, isooctahexacontane, isohexapentacontahectane, isopentacontaoctactane, petrolanum, isopropyl palmitate, isopropyl isostearate or mixture thereof. The composition according to the invention may comprise from 0.1% to 15%, preferably from 1% to 7%, more preferably from 3% to 5% of at least one emollient material by weight of the composition.

Cosmetic compositions according to the present invention may further comprise at least one particulate material. Particulate materials may comprise colored and uncolored pigments, interference pigments, inorganic powders, composition powders, optical brightener particles, or mixture thereof, said material being known in the art. These particulate materials do not comprise inorganic sunscreen active. Preferably, particulate materials comprise free-flowing, solid (i.e. not hollow) materials that are insoluble in both water and oil, with a median particle size of from 0,1 µm to 75 µm, more preferably from 0,2 µm to 30µm, and with a refractive index of from 1.3 to 1.7, said materials being dispersed in the composition. Suitable particulate materials are organic or organosilicone or inorganic. The composition according to the invention may comprise from 0.01 % to 20%, preferably from 0.05% to 10%, and more preferably from 0.1% to 5% particulate materials by weight of the composition.

The oil-in-water emulsion according to the present invention may further comprise a structuring agent to assist in the formation of a liquid crystalline gel network structure. Preferably, structuring agents are selected from stearic acid, palmitic acid, stearyl alcohol, cetyl alcohol, behenyl alcohol, steareth-2, steareth-21, or mixtures thereof The cosmetic composition according to the invention may comprise from 0.5% to 20%, preferably from 1% to 10%, more preferably from 1% to 5% of a structuring agent by weight of the composition.

A variety of additional optional ingredients may be incorporated into the compositions of the present invention. Non-limiting examples of these additional ingredients include additional skin care actives such as bisabolol, dialkanoyl hydroxyproline compounds, farnesol, flavonoids, guanidine (e.g., amino guanidine), N-acyl amino acid compounds, peptides, phytantriol, phytosterols, salicylic compounds, urea as well as compounds such as anti-acne compounds (e.g. resorcinol, erythromycin); antioxidants compounds (e.g., phytosterols, lipoic acid); skin soothing and healing agents; anti-wrinkle/anti-atrophy actives; conditioning agents; anti-inflammatory agents; skin lightening agents; antimicrobial/antibacterial/antifungal actives; chelators and sequestrants; and agents suitable for aesthetic purposes such as essential oils, fragrances, skin sensates, opacifiers, aromatic compounds (e.g., clove oil, menthol, camphor, eucalyptus oil, and eugenol), preservatives, or mixtures thereof.

The composition of the present invention may be useful for treating a number of mammalian keratinous tissue conditions. Such cosmetic treatment of keratinous conditions can include prophylactic and therapeutic regulation. More specifically, such cosmetic composition can be useful for regulating the skin barrier function, the skin tone/eveness, the skin texture, for providing antioxidant effects, etc. For instance, the cosmetic composition of the present invention can be for regulating visible and/or tactile discontinuities in mammalian keratinous tissue, including discontinuities in skin texture and color.

Regulating keratinous tissue conditions can involve topically applying to the keratinous tissue a safe and effective amount of the composition of the present invention. The amount of the composition that is applied, the frequency of application, and the period of use will vary widely depending upon the level of components of the given composition and the level of regulation desired, e.g., in view of the level of keratinous tissue damage present or expected to occur.

### Examples

The compositions according to the present invention detailed below comprise four different phases of components (cf. table below). Phase A and phase B are heated separately at 75°C under agitation at 150rpm. Phase B is then added to A and the obtained mixture is mixed at 13,000 rpm. (using a high shear mixer such as ultra tarax® to emulsify). Said mixture is then cooled to 50°C and phase C is added. The obtained mixture is cooled to 40°C and phase D and then phase E are added.

| **Ingredient** | **Ph** | **1** | **2** | **3** | **4** | **5** | **6** | **7** | **8** |
|---|---|---|---|---|---|---|---|---|---|
| Deionised water | B | Qs | Qs | Qs | Qs | Qs | Qs | Qs | Qs |
| Glycerin | B | 10.0 | 10.0 | 10.0 | 10.0 | 10.0 | 7.0 | 10.0 | 10.0 |
| Octyl methoxycinnamate | A | | | | | | | | 2.00 |
| Octyl Salicylate (Ethylhexyl Salicylate) | A | 4.00 | | | | | | | |
| Avobenxone (Butyl methoxydibenzoylmethane) | A | 2.00 | | | | | | | |
| Octocrylene | A | 1.00 | | | | | | | |
| Phenylbenzimidazole Sulfonic Acid | B | 1.00 | | | | | | | |
| Capric/Caprylic Triglycerides and Sodium Acrylates Copolymer | D | 3.00 | 2.50 | 2.00 | 1.80 | 2.25 | 1.50 | 2.25 | 2.00 |
| Xanthan gum | B | | | | | | 0.06 | | |
| Niacinamide | B | 5.00 | 3.50 | 2.00 | 2.00 | 2.00 | 1.00 | 2.00 | 5.00 |
| D-Panthenol | B | 1.25 | 1.00 | 0.75 | 0.50 | 0.50 | 0.25 | 0.50 | 1.25 |
| Tocopheryl Acetate | A | 0.50 | 0.05 | 0.25 | 0.25 | 0.50 | 0.25 | 0.25 | 0.50 |
| Vitamin C | E | 0.00 | 3.00 | 1.00 | 0.00 | 0.00 | 0.00 | 0.00 | 0.00 |
| Magnesium Ascorbyl Phosphate | E | 0.50 | | | 0.50 | 0.25 | 0.00 | 0.00 | 0.50 |
| Sodium Ascorbyl Phosphate | E | | | | | | 0.50 | 0.05 | |
| Dimethicone and Dimethiconol | C | 2.00 | 1.00 | | 2.00 | 2.00 | 2.00 | 2.00 | 1.50 |
| Dimethicone 350 CS | C | | 1.00 | 0.50 | | | | | 0.50 |
| Cyclopentasiloxane & Dimethicone copyol | C | | | 2.00 | | | | | |
| Petrolatum | A | | | | | 0.10 | | | 1.50 |
| Isopropyl Isostearate | A | | 1.50 | 1.33 | 1.33 | | | 1.33 | |
| Isopropyl palmitate | A | 1.00 | | | | 1.40 | 1.00 | | 1.50 |
| Cetyl Alcohol | A | 1.00 | 0.80 | 0.90 | 0.80 | 0.70 | 1.00 | 0.80 | 2.00 |
| Behenyl Alcohol | A | | | | 0.60 | 0.50 | | | 0.40 |
| Stearyl Alcohol 95% | A | 0.75 | 0.45 | 0.60 | 0.40 | 0.90 | 0.50 | 0.55 | 1.00 |
| Stearic Acid | A | 0.10 | 0.10 | 0.10 | 0.10 | 0.10 | 0.10 | 0.10 | 0.30 |
| Sorbitan Stearate | A | 0.60 | 0.70 | 1.00 | 0.90 | 1.30 | 0.50 | 0.90 | |
| Cetearyl Glucoside & Cetearyl Alcohol | A | 0.40 | 0.10 | 0.20 | 0.30 | 0.05 | 0.50 | 0.10 | |
| Polyethylene | C | 1.00 | 1.00 | 2.00 | 1.50 | 0.20 | | 0.80 | 1.00 |
| Aluminum Starch Octenylsuccinate | C | 0.50 | 1.00 | 2.00 | 0.50 | 0.20 | 1.00 | | |
| CI 77891 titanium dioxide | C | 0.10 | 0.50 | 0.25 | 0.30 | | | | |
| Perfume | E | | | | | | 0.20 | | |
| Disodium EDTA | B | 0.10 | 0.10 | 0.10 | 0.10 | 0.10 | 0.10 | 0.10 | 0.10 |
| PEG-100 Stearate | A | 0.10 | 0.10 | 0.10 | 0.10 | 0.10 | 0.10 | 0.10 | 0.30 |
| Ethylparaben | A | 0.10 | 0.20 | 0.20 | 0.20 | 0.20 | 0.20 | 0.20 | 0.10 |
| Methylparaben | B | 0.20 | | | | | | | 0.20 |
| Propyl Paraben | A | 0.10 | 0.10 | 0.10 | 0.10 | 0.10 | 0.10 | 0.10 | 0.10 |
| Benzyl Alcohol | E | 0.25 | 0.25 | 0.25 | 0.25 | 0.25 | 0.25 | 0.25 | 0.25 |
| Sodium Hydroxide | B | | 0.04 | 0.03 | 0.02 | 0.04 | 0.02 | 0.01 | 0.07 |
| Triethanolamine | B | 0.89 | | | | | | | 0.89 |
| Laureth-7 | A | 0.02 | 0.02 | 0.02 | 0.02 | 0.02 | 0.02 | 0.02 | 0.02 |
| Oleth-3 | A | 0.07 | 0.05 | 0.05 | 0.05 | 0.05 | 0.05 | 0.05 | 0.07 |
| Fruitapone | E | 5.00 | 1.00 | 0.10 | 0.01 | 0.01 | 0.25 | 0.05 | 0.05 |
| Rose Hip Extract | E | 0.10 | 0.10 | 0.10 | 0.10 | | | 0.10 | 0.10 |
| Grape Seed extract | E | | | | | | 0.05 | | |
| Ginseng | E | | | | | | 0.10 | | |
| Lemon Grass | E | | | | | | 0.05 | | |
| Wheat Germ Extract | E | | | | | 0.05 | | | |
| Calendula Extract | E | 0.05 | 0.05 | 0.05 | 0.05 | | | 0.05 | 0.05 |
| Honey | E | | | | | 0.05 | | | |
| Sweet Almond Oil | E | 0.05 | 0.05 | 0.05 | 0.05 | | | 0.05 | 0.05 |
| | | **100** | **100** | **100** | **100** | **100** | **100** | **100** | **100** |

## Claims

1. Cosmetic Topical composition comprising a hydrophilic extract of Seabuckthorn and from 0.01% to 10% of a hydrophilic vitamin component, by weight of the composition.

2. Composition according to claim 1, wherein the hydrophilic vitamin component comprises Vitamin B₃, salts or esters thereof; pro-Vitamin B₅, salts or esters thereof; Vitamin C, salts or esters thereof; hydrophilic derivatives of vitamin E; or mixtures thereof; preferably vitamin B₃, salts or esters thereof; vitamin B₅, salts or esters thereof, or mixtures thereof.

3. Composition according to claim 1 or 2, wherein the hydrophilic vitamin component comprises vitamin B₃, salts or esters thereof, or mixtures thereof

4. Composition according to any one of the preceding claims comprising a combination of a hydrophilic extract of seabuckthorn and niacinamide.

5. Composition according to any one of the preceding claims comprising from 0.0001% to 1%, preferably from 0.001% to 0.4%, and more preferably from 0.002% to 0.15% of a hydrophilic extract of Seabuckthorn by weight of the composition.

6. Composition according to any one of the preceding claims comprising from 0.1% to 9%, preferably from 0.5% to 5% of the hydrophilic vitamin component by weight of the composition

7. Composition according to any one of the preceding claims further comprising a humectant comprising polyhydric alcohol" the polyhydric alcohol preferably comprising glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerin or mixtures thereof.

8. Cosmetic composition according to claim 7 comprising a humectant comprising glycerine.

9. Cosmetic composition according to any one of the preceding claims, further comprising a thickener, preferably comprising C₁₂-C₂₄ fatty acids; C₁₀-C₃₀ fatty alcohols; N-fatty glutamic acid dialylamides; carboxy methyl cellulose and derivatives thereof; polysaccharide gums; starch and derivatives thereof; particulate thickeners; carboxyvinyl polymers; sodium acrylate copolymers and hydrophobically modified sodium acrylate copolymers; polyacrylamide copolymers; polyacrylates; acrylamide/ammonium acrylate copolymer; ammonium acryloyldimethyl taurate and ammonium acryloyldimethyl taurate crosspolymers and copolymers and hydrophobically modified ammonium acryloyldimethyl taurate copolymers; catinionically charged polymers; or mixtures thereof, more preferably comprising a sodium acrylate copolymer.

10. Composition according to any one of the preceding claims further comprising an additional hydrophilic botanical component, preferably an hydrophilic extract of aloe vera leaf, argan tree leaf and/or fruit, baobab or Cream of Tartar tree leaf, bearberry leaf, brahmi whole plant, butcher's broom, centella asiatica, chamomile, chestnut, clary sage leaf, eyebright leaf, ginkgo biloba leaf, grape fruit, leaf and/or seed, green tea leaf, honey, horestail leaf, horse chestnut seed, Indian cress whole plant, lemongrass plant leaf, linden wood, leaf and/or bark, liquorice root, marigold flower, mimosa leaf and/or bark, mulberry leaf, neem leaf and/or bark, nettle leaf oat germ and/or bran, oergano leaf, olive leaf and/or fruit, panax ginseng root extract, plantago leaf, propolis, rose hip fruit, rosemary leaf, sage leaf, st. Mary's thisle, sesame seed, sweet manadarin peel, wheat bran and/or germ, willow bark, witch hazel, or mixtures thereof, more preferably an hydrophilic extract of rose hip fruit, ginko biloba leaf, panax ginseng root or mixtures thereof, still more preferably an hydrophilic extract of rose hip fruit.

11. Composition according to any one of the preceding claims further comprising a hydrophobic botanical component, preferably comprising argan oil, bilberry seed oil, blackcurrant seed oil, cotton seed oil, cranberry seed oil, cumin extract, evening primrose oil, grape seed oil, grapefruit seed oil, kiwi seed oil, lavender, ligonberry seed oil, lime seed oil, linseed oil, liquorice root, olive oil), olive wax, organic grape seed oil, oriental popy seed oil, meadowfoam seed oil, neem leaf and/or bark oil, palm oil, papaya seed oil, passion fruit seed oil, pumpkin seed oil, raspberry seed oil, rosehip seed oil, rosemary oil, seabuckthorn seed oil, sesame seed oil, shea butter, soy extract, sweet almond oil, watermelon seed oil, or mixtures thereof.

12. Composition according to any one of the preceding claims further comprising a hydrophobic vitamin component, preferably comprising vitamin E or hydrophobic derivatives thereof, vitamin D or derivatives thereof, or mixtures thereof

13. Composition according to any one of the preceding claims further comprising a hydrophobic organic sunscreen, preferably alkyl β,β-diphenylacrylate and/or α-cyano β,β-diphenylacrylate derivatives, dibenzoylmethane derivatives, benzophenone derivatives, cinnamic derivatives, salicylic derivatives, camphor derivatives, triazine derivatives, anthranilate derivatives, p-aminobenzoic acid derivatives, or mixture thereof, more preferably alkyl β,β-diphenylacrylate and/or α-cyano β,β-diphenylacrylate derivatives, dibenzoylmethane derivatives, or mixture thereof, still more preferably 2-ethylhexyl 2-cyano-3,3-diphenylacrylate, butyl methoxydibenzoylmethane, or mixture thereof.

14. Composition according to any one of the preceding claims further comprising a hydrophilic organic sunscreen, preferably comprising 2-phenylbenzimidazole-5-sulfonic acid.

15. Composition according to any one of the preceding claims further comprising a tanning active, preferably dihydroxyacetone, salts, derivatives or tautomers thereof, or mixtures thereof.

16. Composition to any one of the preceding claims further comprising at least one sugar amine, preferably glucosamine, N-acetyl glucosamine, mannosamine, N-acetyl mannosamine, galactosamine, N-acetyl galactosamine, isomers thereof, salts thereof, or mixtures thereof.

17. Composition to any one of the preceding claims further comprising at least one hexaminidine compound, salts or derivatives thereof, or mixtures thereof.

18. Cosmetic composition according to any one of the preceding claims, which is in the form of an emulsion, preferably an oil-in-water emulsion.

19. Use of a cosmetic composition according to claims 1 to 18 for the improvement of skin tone.

## Patentansprüche

1. Topische Kosmetikzusammensetzung, umfassend einen hydrophilen Sanddomextrakt und zu 0,01 Gew.-% bis 10 Gew.-% der Zusammensetzung einen hydrophilen Vitaminbestandteil.

2. Zusammensetzung nach Anspruch 1, wobei der hydrophile Vitaminbestandteil Vitamin B₃, Salze oder Ester davon, Provitamin B₅, Salze oder Ester davon, Vitamin C, Salze oder Ester davon, hydrophile Derivate von Vitamin E oder Mischungen davon, vorzugsweise Vitamin B₃, Salze oder Ester davon, Vitamin B₅, Salze oder Ester davon oder Mischungen davon umfasst.

3. Zusammensetzung nach Anspruch 1 oder 2, wobei der hydrophile Vitaminbestandteil Vitamin B₃, Salze oder Ester davon oder Mischungen davon umfasst

4. Zusammensetzung nach einem der vorstehenden Ansprüche, umfassend eine Kobination aus einem hydrophilen Sanddornextrakt und Niacinamid.

5. Zusammensetzung nach einem der vorstehenden Ansprüche, umfassend zu 0,0001 Gew.-% bis 1 Gew.-%, vorzugsweise zu 0,001 Gew.-% bis 0,4 Gew.-% und bevorzugter zu 0,002 Gew.-% bis 0,15 Gew.-% der Zusammensetzung einen hydrophilen Sanddornextrakt.

6. Zusammensetzung nach einem der vorstehenden Ansprüche, umfassend zu 0,1 Gew.-% bis 9 Gew.-%, vorzugsweise zu 0,5 Gew.-% bis 5 Gew.-% der Zusammensetzung den hydrophilen Vitaminbestandteil.

7. Zusammensetzung nach einem der vorstehenden Ansprüche, ferner umfassend ein Feuchthaltemittel, das Polyol umfasst, wobei das Polyol vorzugsweise Glycerin, Propylenglycol, Dipropylenglycol, Polypropylenglycol, Polyethylenglycol, Sorbit, Hydroxypropylsorbit, Hexylenglycol, 1,3-Butylenglycol, 1,2,6-Hexantriol, ethoxyliertes Glycerin, propoxyliertes Glycerin oder Mischungen davon umfasst.

8. Kosmetikzusammensetzung nach Anspruch 7, umfassend ein Feuchthaltemittel, das Glycerin umfasst.

9. Kosmetikzusammensetzung nach einem der vorstehenden Ansprüche, ferner umfassend ein Verdickungsmittel, vorzugsweise umfassend C₁₂-C₂₄-Fettsäuren, C₁₀-C₃₀-Fettalkohole, N-Fettglutaminsäurediallylamide, Carboxymethylcellulose und Derivate davon, Polysaccharid-Gummistoffe, Stärke und Derivate davon, teilchenförmige Verdickungsmittel, Carboxyvinylpolymere, Natriumacrylatcopolymere und hydrophob modifizierte Natriumacrylatcopolymere, Polyacrylamidcopolymere, Polyacrylate, Acrylamid/Ammoniumacrylat-Copolymer, Ammoniumacryloyldimethyltaurat und Ammoniumacryloyldimethyltaurat-Kreuzpolymere und -Copolymere und hydrophob modifizierte Ammoniumacryloyldimethyltaurat-Copolymere, kationisch geladene Polymere oder Mischungen davon, bevorzugter umfassend ein Natriumacrylatcopolymer.

10. Zusammensetzung nach einem der vorstehenden Ansprüche, ferner umfassend einen zusätzlichen hydrophilen pflanzlichen Bestandteil, vorzugsweise einen hydrophilen Extrakt von Aloe-Vera-Blatt, Arganbaumblatt und/oder -frucht, Baobab- oder Affenbrotbaumblatt, Bärentraubenblatt, Brahmi (alle Pflanzenteile), Stechendem Mäusedorn, Tigergras, Kamille, Kastanie, Muskatellersalbeiblatt, Augentrostblatt, Ginkgo-Biloba-Blatt, Grapefruit, -blatt und/oder -samen, Grünteeblatt, Honig, Zinnkrautblatt, Rosskastaniensamen, Kapuzinerkresse (alle Pflanzenteile), Zitronengrasblatt, Lindenholz, -blatt und/oder -rinde, Süßholzwurzel, Ringelblume, Mimosenblatt und/oder -rinde, Maulbeerblatt, Neemölblatt und/oder -rinde, Nesselblatt, Haferkeim und/oder -kleie, Oreganoblatt, Olivenblatt und/oder -frucht, Panax-Ginsengwurzelextrakt, Wegerichblatt, Bienenharz, Hagebuttenfrucht, Rosmarinblatt, Salbeiblatt, Mariendistel, Sesamkorn, Schale süßer Mandarine, Weizenkleie und/oder -keim, Weidenrinde, Zaubernuss oder Mischungen davon, bevorzugter einen hydrophilen Extrakt von Hagebuttenfrucht, Ginkgo-Biloba-Blatt, Panax-Ginsengwurzel oder Mischungen davon, noch bevorzugter einen hydrophilen Extrakt von Hagebuttenfrucht.

11. Zusammensetzung nach einem der vorstehenden Ansprüche, ferner umfassend einen hydrophoben pflanzlichen Bestandteil, vorzugsweise umfassend Arganöl, Moorbeerensamenöl, Schwarze-Johannisbeere-Samenöl, Baumwollsamenöl, Cranberry-Samenöl, Cuminextrakt, Nachtkerzenöl, Weintraubensamenöl, Grapefruitsamenöl, Kiwisamenöl, Lavendel, Preiselbeersamenöl, Limonensamenöl, Leinöl, Süßholzwurzel, Olivenöl, Olivenwachs, organisches Weintraubensamenöl, Türkenmohnsamenöl, Sumpfblumensamenöl, Neemölblatt- und/oder -rindenöl, Palmöl, Papayasamenöl, Maracujasamenöl, Kürbiskernöl, Himbeersamenöl, Hagebuttensamenöl, Rosmarinöl, Sanddornsamenöl, Sesamkornöl, Sheabutter, Sojaextrakt, Süßmandelöl, Wassermelonensamenöl oder Mischungen davon.

12. Zusammensetzung nach einem der vorstehenden Ansprüche, ferner umfassend einen hydrophoben Vitaminbestandteil, vorzugsweise umfassend Vitamin E oder hydrophobe Derivate davon, Vitamin D oder Derivate davon oder Mischungen davon.

13. Zusammensetzung nach einem der vorstehenden Ansprüche, ferner umfassend ein hydrophobes organisches Sonnenschutzmittel, vorzugsweise Alkyl-β,β-diphenylacrylat und/oder α-Cyano-β,β-diphenylacrylat-Derivate, Dibenzoylmethan-Derivate, Benzophenon-Derivate, Zimt-Derivate, Salicyl-Derivate, Campher-Derivate, Triazin-Derivate, Anthranilat-Derivate, p-Aminobenzoesäure-Derivate oder Mischungen davon, bevorzugter Alkyl-β,β-diphenylacrylat und/oder α-Cyano-β,β-diphenylacrylat-Derivate, Dibenzoylmethan-Derivate oder Mischungen davon, noch bevorzugter 2-Ethylhexyl-2-cyano-3,3-diphenylacrylat, Butylmethoxydibenzoylmethan oder Mischungen davon.

14. Zusammensetzung nach einem der vorstehenden Ansprüche, ferner umfassend ein hydrophiles organisches Sonnenschutzmittel, vorzugsweise umfassend 2-Phenylbenzimidazol-5-sulfonsäure.

15. Zusammensetzung nach einem der vorstehenden Ansprüche, ferner umfassend ein Bräunungsmittel, vorzugsweise Dihydroxyaceton, Salze, Derivate oder Tautomere davon oder Mischungen davon.

16. Zusammensetzung nach einem der vorstehenden Ansprüche, ferner umfassend mindestens ein Zuckeramin, vorzugsweise Glucosamin, N-Acetylglucosamin, Mannosamin, N-Acetylmannosamin, Galactosamin, N-Acetylgalactosamin, Isomere davon, Salze davon oder Mischungen davon.

17. Zusammensetzung nach einem der vorstehenden Ansprüche, ferner umfassend mindestens eine Hexaminidin-Verbindung, Salze oder Derivate davon oder Mischungen davon.

18. Kosmetikzusammensetzung nach einem der vorstehenden Ansprüche, die die Form einer Emulsion, vorzugsweise einer Öl-in-Wasser-Emulsion, aufweist.

19. Verwendung einer Kosmetikzusammensetzung nach Ansprüche 1 bis 18 zur Verbesserung des Hauttons.

## Revendications

1. Composition cosmétique topique comprenant un extrait hydrophile d'argousier et de 0,01 % à 10 % d'un composant de vitamine hydrophile, en poids de la composition.

2. Composition selon la revendication 1, dans laquelle le composant de vitamine hydrophile comprend de la vitamine B₃, des sels ou des esters de celle-ci ; de la provitamine B₅, des sels ou des esters de celle-ci ; de la vitamine C, des sels ou des esters de celle-ci ; des dérivés hydrophiles de vitamine E ; ou leurs mélanges ; de préférence, de la vitamine B₃, des sels ou des esters de celle-ci ; de la vitamine B₅, des sels ou des esters de celle-ci, ou leurs mélanges.

3. Composition selon la revendication 1 ou 2, dans laquelle le composant de vitamine hydrophile comprend de la vitamine B₃, des sels ou des esters de celle-ci, ou leurs mélanges.

4. Composition selon l'une quelconque des revendications précédentes, comprenant une combinaison d'un extrait hydrophile d'argousier et de niacinamide.

5. Composition selon l'une quelconque des revendications précédentes, comprenant de 0,0001 % à 1 %, de préférence de 0,001 % à 0,4 %, et plus préférablement de 0,002 % à 0,15 % d'un extrait hydrophile d'argousier en poids de la composition.

6. Composition selon l'une quelconque des revendications précédentes, comprenant de 0,1 % à 9 %, de préférence de 0,5 % à 5 % du composant de vitamine hydrophile en poids de la composition

7. Composition selon l'une quelconque des revendications précédentes, comprenant en outre un humectant comprenant de l'alcool polyhydrique, l'alcool polyhydrique comprenant de préférence de la glycérine, du propylène glycol, du dipropylène glycol, du polypropylène glycol, du polyéthylène glycol, du sorbitol, de l'hydroxypropyl sorbitol, de l'hexylène glycol, du 1,3-butylène glycol, du 1,2,6-hexanetriol, de la glycérine éthoxylée, de la glycérine propoxylée ou leurs mélanges.

8. Composition cosmétique selon la revendication 7, comprenant un humectant comprenant de la glycérine.

9. Composition cosmétique selon l'une quelconque des revendications précédentes, comprenant en outre un épaississant, comprenant de préférence des acides gras en C₁₂ à C₂₄ ; des alcools gras en C₁₀ à C₃₀ ; des N-acide gras glutamique dialylamides ; de la carboxyméthylcellulose et ses dérivés ; des gommes de polysaccharide ; de l'amidon et ses dérivés ; des épaississants particulaires ; des polymères carboxyvinyle ; des copolymères d'acrylate de sodium et des copolymères d'acrylate de sodium rendus hydrophobes ; des copolymères de polyacrylamide ; des polyacrylates ; un copolymère acrylamide/acrylate d'ammonium ; de l'acryloyldiméthyl taurate d'ammonium et des polymères réticulés et copolymères d'acryloyldiméthyl taurate d'ammonium et des copolymères d'acryloyldiméthyl taurate d'ammonium rendus hydrophobes ; des polymères chargés cationiquement ; ou leurs mélanges, plus préférablement comprenant un copolymère d'acrylate de sodium.

10. Composition selon l'une quelconque des revendications précédentes, comprenant en outre un composant botanique hydrophile supplémentaire, de préférence un extrait hydrophile de feuille d'aloé véra, feuille et/ou fruit d'arganier, feuille de baobab ou d'adansonia digitata, feuille de busserole, plante entière de brahmi, fragon épineux, centella asiatica, camomille, châtaigne, feuille de sauge sclarée, feuille d'euphraise, feuille de ginkgo biloba, fruit, feuille et/ou graine de vigne, feuille de thé vert, miel, feuille de prêle, graine de marron d'Inde, plante entière de capucine, feuille de la plante lemon-grass, bois, feuille et/ou écorce de tilleul, racine de réglisse, fleur du souci, feuille et/ou écorce de mimosa, feuille de mûrier, feuille et/ou écorce de margousier, feuille d'ortie, germe et/ou son d'avoine, feuille d'origan, feuille et/ou fruit de l'olivier, extrait de racine de ginseng asiatique, feuille de plantago, propolis, fruit de cynorhodon, feuille de romarin, feuille de sauge, chardon-Marie, graine de sésame, pelure de mandarine sucrée, son et/ou germe de blé, écorce de saule, hamamélis, ou leurs mélanges, plus préférablement un extrait hydrophile de fruit de cynorhodon, feuille de ginko biloba, racine de ginseng asiatique ou leurs mélanges, encore plus préférablement un extrait hydrophile de fruit de cynorhodon.

11. Composition selon l'une quelconque des revendications précédentes comprenant en outre un composant botanique hydrophobe, comprenant de préférence de l'huile d'argan, de l'huile de graines de myrtilles, de l'huile de graines de cassis, de l'huile de graines de coton, de l'huile de graines de coton, de l'extrait de cumin, de l'huile d'oenothère, de l'huile de pépins de raisins, de l'huile de graines de pamplemousses, de l'huile de graines de kiwis, de la lavande, de l'huile de graines d'airelles, de l'huile de graines de limes, de l'huile de lin, une racine de réglisse, de l'huile d'olive, de la cire d'olive, de l'huile de pépins de raisins organique, de l'huile de graines d'oriental poppy, de l'huile de graines d'écume des prés, de l'huile de feuille et/ou d'écorce de margousier, de l'huile de palme, de l'huile de graines de papayes, de l'huile de graines de fruits de la passion, de l'huile de graines de potirons, de l'huile de graines de framboise, de l'huile de graines de cynorhodon, de l'huile de romarin, de l'huile de graines d'argousier, de l'huile de graines de sésame, du beurre de karité, de l'extrait de soja, de l'huile d'amande douce, de l'huile de graines de pastèques, ou leurs mélanges.

12. Composition selon l'une quelconque des revendications précédentes, comprenant en outre un composant de vitamine hydrophobe, comprenant de préférence de la vitamine E ou ses dérivés hydrophobes, de la vitamine D ou ses dérivés, ou leurs mélanges.

13. Composition selon l'une quelconque des revendications précédentes, comprenant en outre un écran solaire organique hydrophobe, de préférence des dérivés d'alkyl β,β-diphényl-acrylate et/ou d'α-cyano β,β-diphényl-acrylate, des dérivés de dibenzoylméthane, des dérivés de benzophénone, des dérivés cinnamiques, des dérivés salicyliques, des dérivés de camphre, des dérivés de triazine, des dérivés d'anthranilate, des dérivés d'acide p-aminobenzoïque, ou mélange de ceux-ci, plus préférablement des dérivés d'alkyl β,β-diphényl-acrylate et/ou α-cyano β,β-diphényl-acrylate, des dérivés de dibenzoylméthane ou mélange de ceux-ci, encore plus préférablement le 2-éthylhexyl 2-cyano-3,3-diphényl-acrylate, le butyl méthoxydibenzoylméthane, ou un mélange de ceux-ci.

14. Composition selon l'une quelconque des revendications précédentes, comprenant en outre un écran solaire organique hydrophile, comprenant de préférence de l'acide 2-phénylbenzimidazole-5-sulfonique.

15. Composition selon l'une quelconque des revendications précédentes, comprenant en outre un agent actif de bronzage, de préférence des sels de dihydroxyacétone, leurs dérivés ou tautomères, ou leurs mélanges.

16. Composition selon l'une quelconque des revendications précédentes, comprenant en outre au moins une amine de sucre, de préférence la glucosamine, la N-acétyl glucosamine, la mannosamine, la N-acétyl mannosamine, la galactosamine, la N-acétyl galactosamine, leurs isomères, leurs sels, ou leurs mélanges.

17. Composition selon l'une quelconque des revendications précédentes, comprenant en outre au moins un composé d'hexaminidine, leurs sels ou dérivés, ou leurs mélanges.

18. Composition cosmétique selon l'une quelconque des revendications précédentes, qui est sous la forme d'une émulsion, de préférence une émulsion huile-dans-eau.

19. Utilisation d'une composition cosmétique selon les revendications 1 à 18, pour l'amélioration du teint.
